# EUROPÄISCHE PATENTSCHRIFT

(11) **EP 1 341 024 B1**
(45) Veröffentlichungstag und Bekanntmachung des Hinweises auf die Patenterteilung: **22.10.2008**
(21) Anmeldenummer: 03003364.1
(22) Anmeldetag: 14.02.2003
(51) Int. Cl.: G02B 21/06, G02B 21/20, A61B 19/00, A61B 3/13

(54) **Beleuchtungseinkoppelung für eine optische Betrachtungseinrichtung**
Illuminating device for optical observation unit
Dispositif d'éclairage pour système optique d'observation

(30) Priorität: 27.02.2002 DE 10208594
(43) Veröffentlichungstag der Anmeldung: 03.09.2003
(73) Patentinhaber: Leica Microsystems (Schweiz) AG, 9435 Heerbrugg (CH)
(72) Erfinder: Sander, Ulrich Dr., 9445 Rebstein (CH)
(74) Vertreter: Rosenich, Paul

(56) Entgegenhaltungen:
- WO-A-95/29419
- WO-A-99/13370
- DE-A- 3 327 672
- US-A- 5 856 883
- US-A- 5 898 518

## Beschreibung

Die Erfindung betrifft ein Stereo-Operationsmikroskop mit einer Beleuchtungseinrichtung nach dem Oberbegriff des Anspruchs 1.

Bei bekannten Beleuchtungseinrichtungen für Operationsmikroskope mit zwei bzw. vier Beobachter-Strahlengängen - vergleiche EP-A1-661 020, DE-A1-43 31 635 und DE-A1-196 50 773 - werden zwei Umlenkelemente für die Einkoppelung des Beleuchtungsstrahlenbündels in die Beobachter-Strahlengänge eingesetzt. Sie sind symmetrisch zu den Haupt-Beobachtungs-Strahlengängen angeordnet, wodurch eine Unsymmetrie in den Assistenten-Beobachter-Strahlengängen entsteht, vergleiche etwa die Fig. 2 in der DE-A1-43 31 635. Die angegebenen Beleuchtungseinrichtungen weisen in der Praxis die folgenden Nachteile hinsichtlich des Rotreflexes auf:
a) Der Hauptbeobachter und der Assistent, die eigentlich das Gleiche sehen sollen, erhalten unterschiedliche Rotreflexe.
b) Da die Beleuchtungsspiegel nur einseitig angeordnet sind, kann beim "Verrollen" eines Patientenauges in seiner Augenhöhle der Rotreflex beider Beobachter verändert werden.

Zwar wurde das Problem bereits früher erkannt (vgl. die US-A-5,856,883, von der der Oberbegriff des Anspruchs 1 ausgeht), doch erfordert die bekannte Lösung eine weitere Beleuchtungsquelle, was den Aufbau komplizierter macht und die Kosten erhöht

Es ist somit Aufgabe der Erfindung, diesen Nachteil bekannter Operationsmikroskope zu beheben und eine Beleuchtungseinkoppelung zu schaffen, die die Beleuchtungs-Strahlenbündel so aufteilt und in einer solchen Art und Weise in die Beobachter-Strahlengänge einkoppelt, dass der Assistent immer den gleichen Rotreflex wie der Haupt-Beobachter sieht

Gelöst wird diese Aufgabe durch die Merkmale des Patentanspruchs 1. Weitere Ausgestaltungen ergeben sich aus den Unteransprüchen.

Zusätzlich zu den beschriebenen Umlenkelementen werden zwei zu der optischen Achse des Hauptobjektivs symmetrisch und kreuzweise angeordnete Umlenkelemente auf den Achsen der Beleuchtungs-Strahlenbündel eingesetzt. Die aus der EP-A1-661 020 bekannten Umlenkelemente werden teildurchlässig und/oder - gemäß einer Weiterentwicklung-in bestimmten Zonen vollreflektierend ausgebildet. Die erfindungsgemäßen zusätzlichen Umlenkelemente sind vollreflektierend. Damit wird eine volle Symmetrie der Beleuchtungseinkoppelung in Relation zu den Beobachter-Strahlengängen des Chirurgen und des Assistenten hergestellt. Der Assistent erhält jetzt im Assistenten-Beobachter-Strahlengang einen gleichwertigen Rotreflex wie der Haupt-Beobachter und der Beobachter erhält beim eingangs erwähnten "Verrollen" des Patientenauges ebenfalls einen guten Rotreflex. Die Nachteile werden also durch die erfindungsgemäße kreuzweise und symmetrische Anordnung der Umlenkelemente kompensiert.

Um bei diesem Aufbau eine weitere Verbesserung zu erzielen, können die beiden erfindungsgemäßen zusätzlichen Umlenkelemente ausklappbar und/oder radial verschiebbar angeordnet sein und/oder mit einem Shutter (Blende) versehen werden. Dadurch werden Nachteile vermieden, die unter Umständen störend wirken können, wenn sich bei der Betrachtung des Patientenauges in erstgenannter erfindungsgemäßer Anordnung die vier Reflexbilder auf der Kornea (Hornhaut des Auges) spiegeln.

Durch die erfindungsgemäße Beleuchtungseinkoppelung mit zwei zusätzlichen, symmetrisch angeordneten Umlenkelementen werden die folgenden Verbesserungen erreicht
- Der Assistent erhält durch die symmetrische Anordnung der eingekoppelten Beleuchtungsstrahlenbündel den gleichen Rotreflex wie der Beobachter.
- Beim "Verrollen" des Patientenauges sehen der Haupt-Beobachter und der Assistent den gleichen Rotreflex.
- Die Beleuchtungspupille wird nicht geometrisch aufgeteilt, sondern über die teildurchlässigen Umlenkelemente im Sinne einer physikalischen Lichtaufteilung auf ein zweites Paar Umlenkelemente geführt. Die Lichtverteilung im Objektfeld ist dadurch homogener.
- Die Bauhöhe des Systems wird durch die physikalische Lichtaufteilung wesentlich geringer gehalten.

Mittels besonderer Weiterentwicklungen der Erfindung lassen sich weitere Verbesserungen erreichen:
- Durch die Shutter (Blenden), beziehungsweise die Ausklappbarkeit der Umlenkelemente können unerwünschte Reflexe vermieden werden.
- Die radiale Verschiebbarkeit der Umlenkelemente ermöglicht es dem Beobachter, die Rotreflexe gemäß seinen Wünschen auszubilden.

Im obigen Text wird zwar auf ein Operationsmikroskop Bezug genommen; die Erfindung ist jedoch nicht darauf eingeschränkt; sie ist vielmehr auch bei anderen optischen Geräten mit einer Beleuchtungseinkoppelung - z.B. Projektoren und Beleuchtungen für Video- und Fotokameras - einsetzbar, wo zwar der Rotreflex keine Bedeutung hat, aber vergleichbare Probleme beim Einspiegeln von Licht auftreten können.

Wenn nachfolgend von der "Achse eines Strahlenbündels" gesprochen wird, ist damit auch das dazugehörige korrelierte Beleuchtungsstrahlenbündel gemeint.

Anhand von schematischen Zeichnungen wird die Erfindung nachfolgend näher erläutert. Es zeigen dabei:
- Fig. 1:: in Schnittsicht den Aufbau einer erfindungsgemäßen Beleuchtungseinkoppelung für ein Mikroskop;
- Fig. 2: in Aufsicht den Aufbau der Beleuchtungseinkoppelung mit den Achsen der Beleuchtungsstrahlenbündel;
- Fig. 3: eine Variante zum Aufbau nach Fig. 2 mit teilweise durchlässigen beziehungsweise verspiegelten Umlenkelementen und
- Fig.4: eine Variante zum Aufbau nach Fig. 2, bei der nur ein Beleuchtungsstrahlenbündel eingesetzt wird.

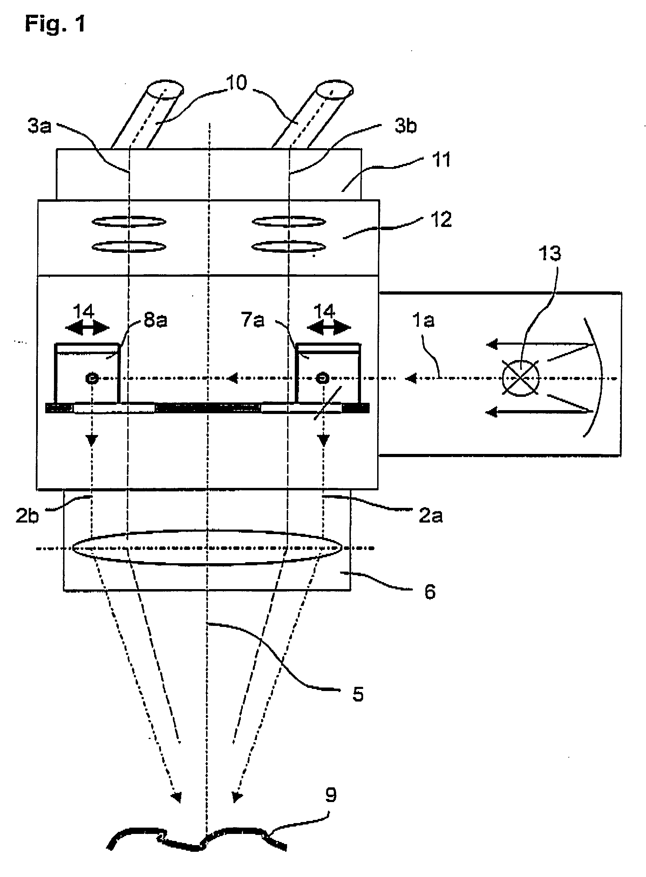

Fig. 1 zeigt in Seitenansicht ein Stereo-Operationsmikroskop mit einer optischen Achse 5 eines Hauptobjektivs 6, mit Okularen 10, einem Tubus 11, einem Zoom 12, mit Beobachterstrahlengängen 3a, 3b und einer Lichtquelle 13. Das von der Lichtquelle 13 erzeugte Strahlenbündel mit seiner Achse 1a wird am teildurchlässigen Umlenkelement 7a anteilmäßig zu einer Achse 2a umgelenkt. Das durchgehende Licht des Beleuchtungsstrahlenbündels wird an einem vollreflektierenden Umlenkelement 8a - beispielsweise einem Spiegel oder einem Prisma - parallel zu einer weiteren Achse 2b umgelenkt.

Die zu den beiden Achsen 2a, 2b korrelierten Strahlenbündel - entstanden aus dem Strahlenbündel 1a nach der Aufteilung und Umlenkung an den Umlenkelementen 7a und 8a - beleuchten über das Hauptobjektiv 6 ein Objektfeld 9. Die beiden radial verschiebbaren Umlenkelemente 7a und 8a - angedeutet durch die Doppelpfeile 14 - befinden sich auf einer Ebene.

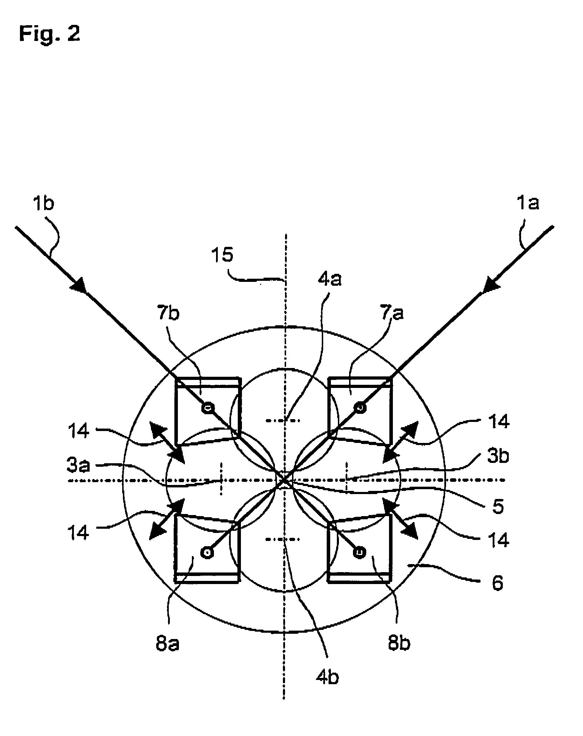

Fig. 2 zeigt in Aufsicht den Aufbau der Beleuchtungseinkoppelung mit der Achse 1a des Beleuchtungsstrahlenbündels, wie in Fig. 1 in Seitenansicht bereits dargestellt Es werden die zu der Achse 1a und den Umlenkelementen 7a und 8a spiegelbildlich angeordnete Achse 1b sowie Umlenkelemente 7b und 8b dargestellt. Die Beobachterstrahlengänge 3a, 3b und Assistenten-Beobachterstrahlengänge 4a, 4b sind in Aufsicht ersichtlich.

Die Achsen 1a bzw. 1 b der Beleuchtungsstrahlenbündel schneiden bei dieser Ausführung die optische Achse 5 des Hauptobjektivs 6.

Zusätzlich ist die - bezüglich der optischen Achse 5 - rotationssymmetrische Anordnung der Umlenkelemente 7a, 8a bzw. 7b, 8b erkennbar.

Die Beleuchtungsstrahlenbündel mit den Achsen 1 a, 1 b werden an den teildurchlässigen Umlenkelementen 7a, 7b in die Beobachterstrahlengänge 3a, 3b und den einen Assistenten-Beobachterstrahlengang 4a eingespiegelt Die durch die Umlenkelemente 7a, 7b durchtretenden Beleuchtungsstrahlenbündel mit den Achsen 1a, 1b werden an den vollreflektierenden Umlenkelementen 8a, 8b in die Beobachterstrahlengänge 3a, 3b, sowie in den zweiten Assistenten-Beobachterstrahlengang 4b eingekoppelt. Durch die zur optischen Achse 5 des Hauptobjektivs 6 symmetrische und kreuzweise Anordnung der Umlenkelemente 7a, 7b und 8a, 8b entsteht eine homogene Beleuchtung des Objektfeldes 9 sowohl für die Beobachterstrahlengänge 3a, 3b wie auch für die Assistenten-Beobachterstrahlengänge 4a, 4b.

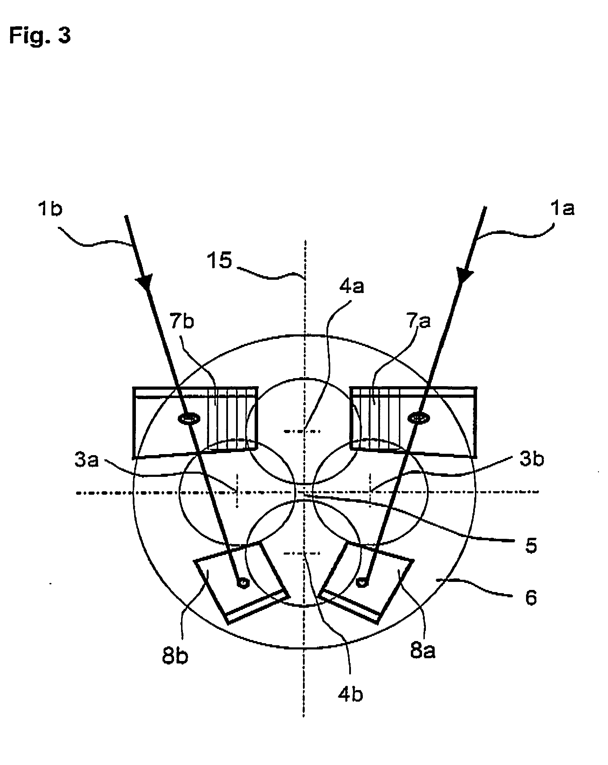

Fig. 3 zeigt als Variante zum Aufbau gemäß Fig. 2 einen Aufbau, bei dem der Schnittpunkt der Achsen 1a, 1 b der Beleuchtungsstrahlengänge von der optischen Achse 5 des Hauptobjektivs 6 abweicht und die Umlenkelemente 7a, 7b in mehrere Zonen - angedeutet durch enge parallele Linien - aufgeteilt sind. Diese Zonen sind durchlässig und/oder teildurchlässig und/oder vollreflektierend ausgebildet. Dadurch ist eine in bestimmten Zonen ungehinderte Transmission der Beleuchtungsstrahlenbündel mit den Achsen 1a, 1b auf die erfindungsgemäßen zusätzlichen Umlenkelemente 8a, 8b möglich. Damit wird ebenso eine weitgehend homogene Beleuchtung über das Objektfeld 9 erreicht.

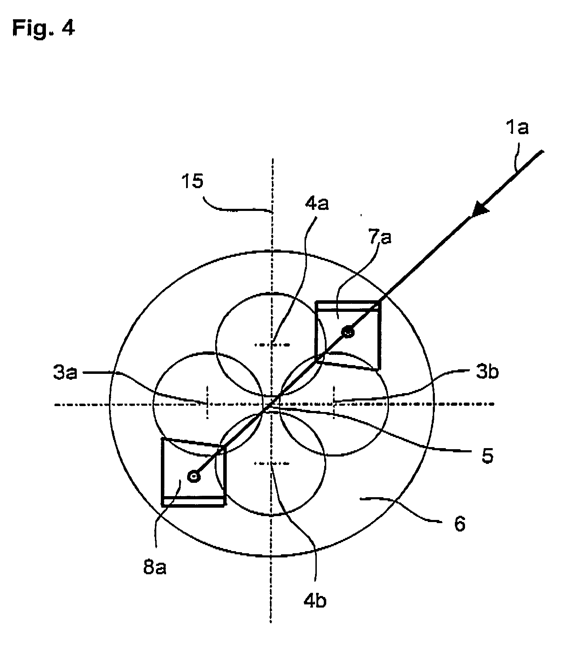

Fig. 4 zeigt eine Variante zum Aufbau gemäß Fig. 2, bei der nur ein einziges Beleuchtungsstrahlenbündel 1a eingesetzt wird. Dadurch können die Kornea-Reflexe (Rotreflex) bei trotzdem gleichmäßiger Ausleuchtung der Beobachterstrahlengänge 3a, 3b und der Assistenten-Beobachterstrahlengänge 4a, 4b ebenso reduziert sowie der konstruktive Aufbau weiter vereinfacht werden.

In den Fällen, bei denen zwei Beleuchtungsachsen angewendet werden, können diese mit zwei separaten Lichtquellen oder mit nur einer Lichtquelle und entsprechenden zusätzlichen Teilerelementen - wie z.B. in der EP-A1-661 020 beschrieben - verwendet werden.

### Bezugszeichenliste

- 1: Achse des Beleuchtungsstrahlenbündels (a, b)
- 2: Achsen der umgelenkten Beleuchtungsstrahlenbündel (a, b)
- 3: Haupt-Beobachterstrahlengänge (a, b)
- 4: Assistenten-Beobachterstrahlengänge (a, b)
- 5: optische Achse des Hauptobjektivs
- 6: Hauptobjektiv
- 7: Umlenkelemente, teildurchlässig (a, b)
- 8: Umlenkelemente, vollreflektierend (a, b)
- 9: Objektfeld
- 10: Okulare
- 11: Tubus
- 12: Zoom
- 13: Lichtquelle
- 14: Doppelpfeil(e) (Verschiebbarkeit der Umlenkelemente (7a, 7b; 8a, 8b))
- 15: Symmetrieachse

## Patentansprüche

1. Stereo-Operationsmikroskop mit um die optische Achse (5) eines Hauptobjektivs (6) angeordneten Hauptbeobachter-Strahlengängen (3a, 3b) und Assistenten-Beobachter-Strahlengängen (4a, 4b),
**gekennzeichnet durch**
symmetrisch
zu einer die optische Achse (5) des Hauptobjektivs (6) senkrecht schneidenden Achse (15), oder symmetrisch
zur optischen Achse (5) des Hauptobjektivs (6)
angeordnete Umlenkelemente (7a, 7b, 8a, 8b),
die im Betriebszustand mindestens ein jeweils entlang einer Achse (1a, 1b) verlaufendes Beleuchtungsstrahlenbündel in jeweils zwei Beleuchtungsstrahlenbündel mit Beleuchtungs-Achsen (2a, 2b) aufteilen,
wobei das in Beleuchtungsrichtung erste Umlenkelement (7a) als teildurchlässiges und/oder teilweise vollverspiegeltes Umlenkelement und das andere - dem Strahlenverlauf des Beleuchtungsstrahlenbündels mit der Achse (1a) folgend - als vollverspiegeltes Umlenkelement (8a) ausgebildet ist,
so dass für einen Hauptbeobachter **durch** die Hauptbeobachter-Strahlengänge (3a, 3b) und einen Assistentenbeobachter **durch** die Assistenten-Beobachter-Strahlengänge (4a, 4b) der gleiche Beleuchtungseffekt hergestellt wird, dass nämlich der Assistenbeobachter und der Hauptbeobachter immer den gleichen Rotreflex sehen.

2. Stereo-Operationsmikroskop nach Anspruch 1, **dadurch gekennzeichnet, dass** vier Umlenkelemente vorgesehen sind, die kreuzweise zur Achse (5) des Hauptobjektivs (6) angeordnet sind, wobei die ersten beiden als teildurchlässige und/oder teilweise vollverspiegelte Umlenkelemente (7a, 7b), und die beiden anderen - ihrem jeweiligen Strahlenverlauf folgend - als vollverspiegelte Umlenkelemente (8a, 8b) ausgebildet sind.

3. Stereo-Operationsmikroskop nach einem der vorhergehenden Ansprüche,
**dadurch gekennzeichnet, dass** die Umlenkelemente (7a, 7b) und (8a, 8b) in einer horizontalen Ebene angeordnet sind.

4. Stereo-Operationsmikroskop nach einem der vorhergehenden Ansprüche,
**dadurch gekennzeichnet, dass** die Umlenkelemente (7a, 7b) und (8a, 8b) als Umlenkspiegel oder Umlenkprismen ausgebildet sind.

5. Stereo-Operationsmikroskop nach Anspruch 2,
**dadurch gekennzeichnet, dass** die beiden Achsen (1a, 1b) der Beleuchtungsstrahlenbündel die optische Achse (5) des Hauptobjektivs (6) in einem Punkt schneiden.

6. Stereo-Operationsmikroskop nach Anspruch 2,
**dadurch gekennzeichnet, dass** der Schnittpunkt der Achsen (1a, 1 b) der Beleuchtungsstrahlenbündel seitlich der optischen Achse (5) des Hauptobjektivs (6) liegt.

7. Stereo-Operationsmikroskop nach einem der Ansprüche 2, 5 oder 6,
**dadurch gekennzeichnet, dass** die ersten beiden Umlenkelemente (7a, 7b) der Beleuchtungsstrahlenbündel als teildurchlässige Umlenkspiegel oder Umlenkprismen ausgebildet sind.

8. Stereo-Operationsmikroskop nach einem der Ansprüche 2, 5 oder 6,
**dadurch gekennzeichnet, dass** die ersten beiden Umlenkelemente (7a, 7b) Zonen mit unterschiedlicher Reflexion beziehungsweise Transmission aufweisen.

9. Stereo-Operationsmikroskop nach einem der Ansprüche 7 oder 8
**dadurch gekennzeichnet, dass** die beiden anderen Umlenkelemente (8a, 8b) als vollreflektierende Umlenkspiegel oder Umlenkprismen ausgebildet sind.

10. Stereo-Operationsmikroskop nach einem der vorhergehenden Ansprüche,
**dadurch gekennzeichnet, dass** zur wahlweisen Abdunkelung der Umlenkelemente (7a, 7b; 8a, 8b) Shutter einsetzbar sind.

11. Stereo-Operationsmikroskop nach einem der Ansprüche 7-10,
**dadurch gekennzeichnet, dass** die beiden anderen Umlenkelemente (8a, 8b) vollständig ausklappbar oder ausschiebbar sind.

12. Stereo-Qperationsmikroskop nach einem der Ansprüche 7-11,
**dadurch gekennzeichnet, dass** die beiden anderen Umlenkelemente (8a, 8b) zur Erzielung unterschiedlicher Beleuchtungswinkel des Objektfeldes (9) verschiebbar angeordnet sind.

13. Stereo-Operationsmikroskop nach einem der Ansprüche 7-12,
**dadurch gekennzeichnet, dass** die beiden anderen Umlenkelemente (8a, 8b) in Form eines einzigen baulichen Elements ausgebildet sind.

14. Stereo-Operationsmikroskop nach einem der vorhergehenden Ansprüche,
**dadurch gekennzeichnet, dass** die äußere Form der Umlenkelemente (7a, 7b und 8a, 8b) der jeweiligen Kontur des Strahlenbündel-Querschnitts der Beobachterstrahlengänge (3a, 3b) und (4a, 4b) angepasst ist

## Claims

1. Surgical stereomicroscope with main observer beam paths (3a, 3b) and assistant observer beam paths (4a, 4b) which are located around the optical axis (5) of a main objective (6), **characterized by** deflection elements (7a, 7b, 8a, 8b) which are arranged symmetrically with respect to an axis (15) which intersects the optical axis (5) of the main objective (6) at right angles or symmetrically with respect to the optical axis (5) of the main objective (6), which split in the operational state at least one illumination beam bundle, which travels in each case along an axis (1a, 1b) into in each case two illumination beam bundles with illumination axes (2a, 2b), wherein the first deflection element (7a) in the illumination direction is in the form of a partially transparent and/or in part fully reflective deflection element and the other one - which follows the beam route of the illumination beam bundle with the axis (1a) - is in the form of a fully reflective deflection element (8a), such that the same illumination effect is produced for a main observer via the main observer beam paths (3a, 3b) and for an assistant observer via the assistant observer beam paths (4a, 4b), to be precise that the assistant observer and the main observer always see the same red reflex.

2. Surgical stereomicroscope according to Claim 1, **characterized in that** four deflection elements are provided which are arranged in the manner of a cross with respect to the axis (5) of the main objective (6), wherein the first two are in the form of partially transparent and/or in part fully reflective deflection elements (7a, 7b) and the other two - following their respective beam route - are in the form of fully reflective deflection elements (8a, 8b).

3. Surgical stereomicroscope according to one of the preceding claims, **characterized in that** the deflection elements (7a, 7b) and (8a, 8b) are arranged in one horizontal plane.

4. Surgical stereomicroscope according to one of the preceding claims, **characterized in that** the deflection elements (7a, 7b) and (8a, 8b) are in the form of deflection mirrors or deflection prisms.

5. Surgical stereomicroscope according to Claim 2, **characterized in that** the two axes (1a, 1b) of the illumination beam bundles intersect the optical axis (5) of the main objective (6) in one point.

6. Surgical stereomicroscope according to Claim 2, **characterized in that** the point of intersection of the axes (1a, 1b) of the illumination beam bundles is located to the side of the optical axis (5) of the main objective (6).

7. Surgical stereomicroscope according to one of Claims 2, 5 or 6, **characterized in that** the first two deflection elements (7a, 7b) of the illumination beam bundles are in the form of partially transparent deflection mirrors or deflection prisms.

8. Surgical stereomicroscope according to one of Claims 2, 5 or 6, **characterized in that** the first two deflection elements (7a, 7b) have zones with different reflection or transmittance.

9. Surgical stereomicroscope according to either of Claims 7 and 8, **characterized in that** the other two deflection elements (8a, 8b) are in the form of fully reflective deflection mirrors or deflection prisms.

10. Surgical stereomicroscope according to one of the preceding claims, **characterized in that** shutters can be used for optionally shading the deflection elements (7a, 7b; 8a, 8b).

11. Surgical stereomicroscope according to one of Claims 7-10, **characterized in that** the other two deflection elements (8a, 8b) can be completely folded out or slid out.

12. Surgical stereomicroscope according to one of Claims 7-11, **characterized in that** the other two deflection elements (8a, 8b) are arranged such that they can be displaced in order to achieve different illumination angles of the object field (9).

13. Surgical stereomicroscope according to one of Claims 7-12, **characterized in that** the other two deflection elements (8a, 8b) are designed in the form of a single structural element.

14. Surgical stereomicroscope according to one of the preceding claims 7-9, **characterized in that** the outer shape of the deflection elements (7a, 7b and 8a, 8b) are matched to the respective outline of the beam bundle cross section of the observer beam paths (3a, 3b) and (4a, 4b).

## Revendications

1. Microscope stéréoscopique d'opération avec des trajets de faisceaux d'observation principaux (3a, 3b) disposés autour de l'axe optique (5) d'un objectif principal (6) et des trajets de faisceaux d'observation d'assistants (4a, 4b),
**caractérisé par le fait que** des éléments de déviation (7a, 7b, 8a, 8b), qui en fonctionnement,
symétriquement par rapport à un axe (15) coupant perpendiculairement l'axe optique (5) de l'objectif principal (6),
ou symétriquement par rapport à l'axe optique (5) de l'objectif principal (6),
divisent au moins un faisceau lumineux d'éclairage se propageant respectivement le long d'un axe (1a, 1b) en respectivement deux faisceaux lumineux d'éclairage ayant les axes d'éclairage (2a, 2b),
le premier élément de déviation (7a) dans la direction de l'éclairage étant réalisé sous la forme d'un élément de déviation partiellement transparent et/ou en partie totalement réfléchissant et l'autre, en suivant la propagation du faisceau lumineux d'éclairage d'axe (1a), étant réalisé sous la forme d'un élément de déviation (8a) totalement réfléchissant,
de manière à fournir le même effet d'éclairage à un observateur principal regardant à travers les trajets de faisceaux d'observation principaux (3a, 3b) et à un observateur assistant regardant à travers les trajets de faisceaux d'observation d'assistants (4a, 4b), pour qu'en l'occurrence l'observateur assistant et l'observateur principal voient toujours le même reflet rouge.

2. Microscope stéréoscopique d'opération selon la revendication 1, **caractérisé en ce qu'**on prévoit quatre éléments de déviation qui sont disposés en croix par rapport à l'axe (5) de l'objectif principal (6), les deux premiers étant réalisés sous la forme d'éléments de déviation partiellement transparents et/ou en partie totalement réfléchissants (7a, 7b) et les deux autres, suivant la propagation de leur faisceau respectif, étant réalisés sous la forme d'éléments de déviation (8a, 8b) totalement réfléchissants.

3. Microscope stéréoscopique d'opération selon une des revendications précédentes, **caractérisé en ce que** les éléments de déviation (7a, 7b) et (8a, 8b) sont disposés dans un plan horizontal.

4. Microscope stéréoscopique d'opération selon une des revendications précédentes, **caractérisé en ce que** les éléments de déviation (7a, 7b) et (8a, 8b) sont réalisés sous la forme de miroirs de déviation ou de prismes de déviation.

5. Microscope stéréoscopique d'opération selon la revendication 2, **caractérisé en ce que** les deux axes (1a, 1b) des faisceaux lumineux d'éclairage coupent l'axe optique (5) de l'objectif principal (6) en un point.

6. Microscope stéréoscopique d'opération selon la revendication 2, **caractérisé en ce que** le point d'intersection des axes (1a, 1b) des faisceaux lumineux d'éclairage se situe latéralement à l'axe optique (5) de l'objectif principal (6).

7. Microscope stéréoscopique d'opération selon une des revendications 2, 5 ou 6, **caractérisé en ce que** les deux premiers éléments de déviation (7a, 7b) des faisceaux lumineux d'éclairage sont réalisés sous la forme de miroirs de déviation ou de prismes de déviation partiellement transparents.

8. Microscope stéréoscopique d'opération selon une des revendications 2, 5 ou 6, **caractérisé en ce que** les deux premiers éléments de déviation (7a, 7b) présentent des zones de réflexion respectivement de transmission différentes.

9. Microscope stéréoscopique d'opération selon une des revendications 7 ou 8**, caractérisé en ce que** les deux autres éléments de déviation (8a, 8b) sont réalisés sous la forme de miroirs de déviation ou de prismes de déviation totalement réfléchissants.

10. Microscope stéréoscopique d'opération selon une des revendications précédentes, **caractérisé en ce qu'**on peut insérer des obturateurs pour occulter au choix les éléments de déviation (7a, 7b ; 8a, 8b).

11. Microscope stéréoscopique d'opération selon une des revendications 7 - 10, **caractérisé en ce que** les deux autres éléments de déviation (8a, 8b) peuvent être totalement basculés ou coulissés vers l'extérieur.

12. Microscope stéréoscopique d'opération selon une des revendications 7 - 11, **caractérisé en ce que** les deux autres éléments de déviation (8a, 8b) sont disposés de manière coulissante pour obtenir différents angles d'éclairage du champ de l'objet (9).

13. Microscope stéréoscopique d'opération selon une des revendications 7 - 12, **caractérisé en ce que** les deux autres éléments de déviation (8a, 8b) sont réalisés sous la forme d'un élément unique de structure.

14. Microscope stéréoscopique d'opération selon une des revendications précédentes, **caractérisé en ce que** la forme extérieure des éléments de déviation (7a, 7b et 8a, 8b) est adaptée au contour respectif de la section transversale du faisceau lumineux des trajets de faisceaux d'observation (3a, 3b) et (4a, 4b).
